# EUROPEAN PATENT APPLICATION

(11) **EP 3 808 327 A1**
(43) Date of publication of application: **21.04.2021**
(21) Application number: 19382896.9
(22) Date of filing: 14.10.2019
(51) Int. Cl.: A61K 8/27, A61Q 11/00, A61K 8/34, A61K 8/60, A61K 8/63

(54) **ORAL CARE COMPOSITON**

(71) Applicant: Lacer, S.A., 08025 Barcelona (ES)
(72) Inventor: MATA MOLINER, Montserrat, E-08025 Barcelona (ES); FERRER SISO, Alicia, E-08025 Barcelona (ES); DEL CASTILLO NIETO, Juan Carlos, E-08025 Barcelona (ES); MIRA OTAL, Javier, E-08025 Barcelona (ES); WIEDEMANN, Ralf, E-08025 Barcelona (ES)
(74) Representative: ABG Intellectual Property Law, S.L.

(57) **Abstract**

The present invention relates to oral care composition comprising a zinc salt having a solubility in water higher than 3.0·10⁴ mg/L at 25 °C, an isopropylmethylphenol compound, a glycyrrhizin derivative and water, as well as to their use in oral care, in particular for the treatment and/or prevention of dental plaque formation, caries formation, calculus formation, halitosis, gingivitis, periodontitis, peri-implant mucositis and/or periimplantitis.

## Description

### FIELD OF THE INVENTION

The invention relates to the field of oral care compositions, in particular to mouthwashes, tooth gels and toothpastes comprising zinc salts having a solubility in water higher than 3.0·10⁴ mg/L at 25 °C, glyzyrrhicin derivatives and isopropylmethylphenols.

### BACKGROUND OF THE INVENTION

Oral hygiene is the practice of keeping the mouth clean, and is considered to be the best means of prevention of cavities (dental caries), gingivitis, periodontitis, and other dental and oral disorders such as bad breath (halitosis). Oral hygiene is necessary for all persons to maintain the health of their teeth and mouth. There are three key steps to regular dental health and maintenance: brushing, flossing and rinsing with a mouthwash. Teeth brushing involves the use of a toothpaste in conjunction with a toothbrush to help remove food debris and dental plaque. Mouthwashes are used for cleaning and freshening the mouth and for oral hygiene. Antiseptic and anti-plaque compounds are present in mouthwashes and toothpaste to kill germs that cause plaque, gingivitis, and bad breath.

Zinc salts are widely used in oral healthcare products, such as toothpastes and mouthwashes, due to their antimicrobial properties against different bacteria, which helps control the development of dental plaque, calculus formation, bleeding gums, and malodour [Fatima, T. et al., J. Pak. Med. Assoc., 2016, 66(8), 1019-1023; Davies, R. M., Periodontology 2000, 2008, 48, 23-30]. Zinc salts do also reduce the breath concentrations of volatile sulphur compounds (VSC), which are associated with halitosis, as well as having direct and indirect inhibitory effects on anaerobe mediated-VSC production [Young, A. et al., Eur. J. Oral Sci., 2002, 110(1), 31-34]. Therefore, the inclusion of said salts in oral care products is highly desirable. In fact, several oral care compositions comprising zinc salts have bend disclosed or are available in the market.

Other compounds having antibacterial effect that can be used in oral care products are isopropylmethylphenols. These compounds penetrate deep-inside plaque and show high bactericidal effect [Park, Y.-D. et al., Int. J. Clin. Prev. Dent., 2010, 6(2), 55-61].

It has also been reported that combinations of zinc salts and o-cymen-5-ol, a particular isopropylmethylphenol compound, have shown direct antimicrobial effect and inhibition of oral disease-related processes and are highly desirable for incorporation in oral care products due to synergic effects against anaerobes [Pizzey, R. L., Int. Dent. J., 2011, 61 (Suppl. 3), 33-40].

Sensory-related aspects are an important contribution to consumers' acceptance and preference of oral care products. Examples of relevant sensory-related aspects for oral care products are appearance, sensory smoothness and granularity, and taste. Despite the beneficial properties of zinc compounds in oral care, zinc salts show a limited solubility in water, which may limit their use in oral care products or provide products having unsatisfactory sensory-related aspects. Thus, there is a need for alternative oral care products comprising zinc salts, in particular in combination with isopropylmethylphenols, and having adequate sensory-related aspects.

### SUMMARY OF THE INVENTION

The present inventors have surprisingly found that glycyrrhizin derivatives improves solubility of zinc salts in oral care compositions, and also when said compositions additionally comprise an isopropylmethylphenol compound. This improvement in solubility allows obtaining clear compositions. The compositions of the invention are also advantageous due to their low cytotoxicity, antioxidative effects, anti-inflammatory effects, wound healing properties, microbiological profiles, cleaning properties, plaque penetration and plaque reduction properties, their properties with respect to their stability, in particular against light, temperature, or external forces such as gravity forces.

Thus, in a first aspect, the present invention relates to an oral care composition comprising:
- a zinc salt having a solubility in water higher than 3.0·10⁴ mg/L at 25 °C,
- an isopropylmethylphenol compound,
- a glycyrrhizin derivative selected from the group consisting of glycyrrhizinic acid, glycyrrhetinic acid, salts thereof, and mixtures thereof, and
- water.

In a second aspect, the present invention relates to an oral care composition as defined in the first aspect for use in the prevention of dental plaque formation, caries formation, calculus formation, halitosis, gingivitis, periodontitis, peri-implant mucositis and/or periimplantitis.

### DETAILED DESCRIPTION OF THE INVENTION

In a first aspect, the present invention relates to an oral care composition comprising:
- a zinc salt having a solubility in water higher than 3.0·10⁴ mg/L at 25 °C,
- an isopropylmethylphenol compound,
- a glycyrrhizin derivative selected from the group consisting of glycyrrhizinic acid, glycyrrhetinic acid, salts thereof, and mixtures thereof, and
- water.

The compositions of the invention are "oral care" compositions, which means that they are intended for topical use in the mouth, so any component present in the composition needs to be orally acceptable, that is, safe for topical use in the mouth in the amounts and concentrations provided.

The term "zinc salt" refers to an ionic compound formed by a zinc cation (Zn²⁺) and an anion. The anion may be monovalent or divalent, organic or inorganic. Where the anion is monovalent the zinc salt is formed by two anions and one zinc cation. Where the anion is divalent, the zinc salt is formed by one anion and one zinc cation. Examples of inorganic anions are chloride (Cl⁻) and sulphate (SO₄²⁻). Examples of organic anions are gluconate, citrate and acetate. The zinc salt may be in anhydrous form or in the form of a hydrate such as the monohydrate or the heptahydrate. The zinc salt of the compositions of the present invention has a solubility in water higher than 3.0·10⁴ mg/L at 25 °C. In a particular embodiment, the zinc salts of the present invention have a solubility in water not higher than 5.0·10⁷ mg/L at 25°C.

Examples of zinc salts having a solubility in water higher than 3.0·10⁴ mg/L at 25 °C are zinc chloride (4.32·10⁶ mg/L at 25 °C), zinc gluconate (1·10⁶ mg/L at 25 °C), zinc sulphate heptahydrate (9.6·10⁵ mg/L at 25 °C), zinc sulphate (5.77·10⁵ mg/L at 25 °C), zinc acetate (4.0·10⁴ mg/L at 25 °C) and zinc citrate (4.0·10⁴ mg/L at 25 °C).

In a preferred embodiment, the zinc salt having a solubility in water higher than 3.0·10⁴ mg/L at 25 °C is selected from the group consisting of zinc chloride, zinc sulphate, zinc gluconate, zinc citrate, zinc acetate and mixtures thereof; preferably zinc chloride, zinc sulphate and zinc gluconate and mixtures thereof; more preferably zinc chloride.

In a preferred embodiment, the zinc salt having a solubility in water higher than 3.0·10⁴ mg/L at 25 °C is present in the oral care compositions of the invention in a concentration of from 0.01 to 1.5 wt% with respect to the total weight of the composition, preferably from 0.05 to 1 wt%, more preferably from 0.05 to 0.7 wt%.

The term "isopropylmethylphenol compound" refers to compounds having a phenyl ring substituted by one hydroxyl group, one methyl group and one isopropyl group, as represented by formula (I).

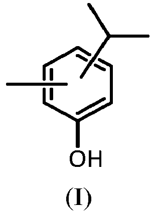

Examples of isopropylmethyl phenol compounds are *o*-cymen-5-ol (or 4-isopropyl-3-methylphenol of formula Ia), thymol (or 2-isopropyl-5-methylphenol of formula Ib) and 3-isopropyl-5-methylphenol (of formula Ic), whose structures are depicted below.

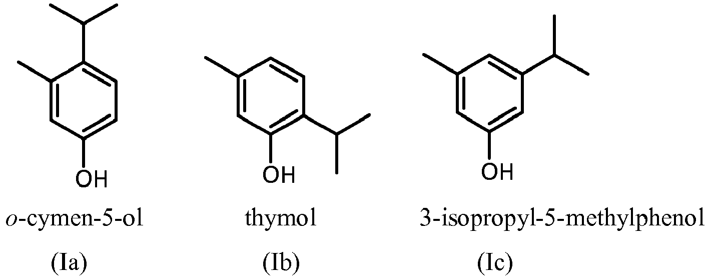

The oral care compositions of the invention may comprise one or more (such as 2 or 3) isopropylmethylphenol compounds, preferably one.

In a preferred embodiment, the isopropylphenylphenol compound is o-cymen-5-ol.

In a preferred embodiment, the isopropylmethylphenol compound is present in the oral care compositions of the invention in a concentration of from 0.005 to 1 wt% with respect to the total weight of the composition, preferably from 0.01 to 0.5 wt%, more preferably from 0.02 to 0.1 wt%.

The glycyrrhizin derivative present in the compositions of the inventions is selected from the group consisting of glycyrrhizinic acid, glycyrrhetinic acid, salts thereof, and mixtures thereof. These compounds are widely applied as a natural sweeteners, and utilized in cosmetics and pharmaceuticals. They can be isolated from the roots and stolon of perennial plants of *Glycyrrhiza* species, such as *Glycyrrhiza glabra.*

"Glycyrrhizinic acid" or "glycyrrhizic acid" has the chemical structure depicted below.

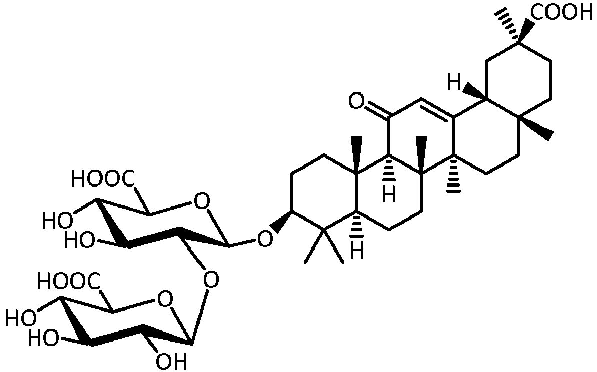

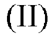

Glycyrrhizinic acid has three acid groups which may form salts with a cation, including organic cations (such as ammonium) as well as inorganic cations (such as alkali metals, e.g. sodium and potassium; and alkaline earth metals, e.g. calcium and magnesium). Preferred cations for forming salts with glycyrrhizinic acid are potassium, sodium and ammonium. Examples of glycyrrhizinic acid salts are dipotassium glycyrrhizinate, diammonium glycyrrhizinate, disodium glycyrrhizinate, monoammonium glycyrrhizinate, monosodium glycyrrhizinate, and monopotassium glycyrrhizinate, preferably dipotassium glycyrrhizinate.

"Glycyrrhetinic acid" or "enoxolone" has the chemical structure depicted below.

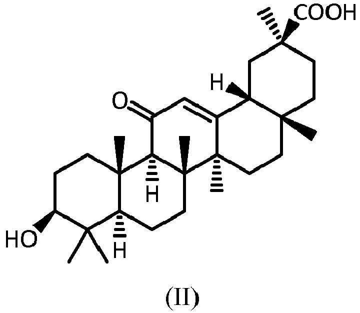

Glycyrrhetinic acid has one acid group which may form salts with a cation, including organic cations (such as ammonium) as well as inorganic cations (such as alkali metals, e.g. sodium and potassium; and alkaline earth metals, e.g. calcium and magnesium). Preferred cations for forming salts with glycyrrhetinic acid are potassium, sodium and ammonium. Examples of glycyrrhetinic acid salts are potassium glycyrrhetinate, ammonium glycyrrhetinate, and sodium glycyrrhetinate.

In a preferred embodiment, the glycyrrhizin derivative in the oral care compositions of the invention is glycyrrhizinic acid or a salt thereof, more preferably dipotassium glycyrrhizinate.

In a preferred embodiment, the glycyrrhizin derivative is present in the oral care compositions of the invention in a concentration of from 0.005 to 1.5 wt% with respect to the total weight of the composition, preferably from 0.01 to 1.5 wt%, more preferably from 0.02 to 1 wt%, more preferably from 0.02 to 0.5 wt%.

In another preferred embodiment of the oral care compositions of the invention, the weight ratio of the zinc salt having a solubility in water higher than 3.0·10⁴ mg/L at 25 °C to the glyzyrrhizin derivative is from 5:1 to 1:1, preferably from 3.5:1 to 1.5:1, more preferably from about 3:1 to about 2:1.

The oral care compositions of the invention also comprise water. Preferably, water is present in an amount of at least 10 wt%, at least 15 wt%, at least 20 wt%, at least 25 wt%, at least 30 wt%, at least 40 wt%, at least 50 wt%, at least 60 wt%, at least 70 wt%, at least 75 wt%, or at least 80 wt%, wherein the wt% is expressed with respect to the total weight of the composition.

In a preferred embodiment, the oral care composition of the invention also comprise one or more ingredients selected from the group consisting of fluoride ion sources, surfactants, co-solvents, humectants, sweeteners, binders, mouth-feel agents, abrasives, desensitizing agents, other oral care active ingredients, pH modifying agents, chelating agents, preservatives, flavoring agents, and colorants.

Fluoride ion sources include sodium fluoride, potassium fluoride, stannous fluoride, potassium monofluorophosphate, sodium monofluorophosphate, ammonium monofluorophosphate, sodium fluorosilicate, ammonium fluorosilicate, amine fluoride such as (N'-octadecyltrimethylendiamine-N,N,N'-tris(2-ethanol)-dihydrofluoride), ammonium fluoride, and combinations thereof. Preferably, the oral care composition of the invention may also contain a source of fluoride ions or fluorine-providing ingredient in amounts sufficient to supply 50 to 5000 ppm fluoride ion, e.g., from 100 to 1000 ppm fluoride ion. Fluoride ion sources may be added to the compositions of the invention at a level in the range of 0.001 wt% to 10 wt%, e.g., from 0.003 wt% to 5 wt%, or from 0.01 wt% to 1.2 wt%, wherein the wt% is expressed with respect to the total weight of the composition. However, it is to be understood that the weights of fluoride salts to provide the appropriate level of fluoride ion will obviously vary based on the weight of the counter ion in the salt, and one of skill in the art may readily determine such amounts. Preferred fluoride ion sources are sodium fluoride and sodium monofluorophsphate.

The oral care compositions of the present invention may optionally comprise a surfactant. The term "surfactant" refers to compounds that are capable of lowering the surface tension. Suitable surfactants include non-ionic, anionic, cationic and amphoteric surfactants. Any orally acceptable surfactant can be used.

Examples of non-ionic surfactants are block copolymers such as ethylene oxide and propylene oxide block copolymers (e.g. poloxamers), ethoxylated hydrogenated castor oils, polyoxyethylene sorbitan esters (e.g. polysorbates), fatty alcohol ethoxylates, alkylphenol ethoxylates, tertiary amine oxides, tertiary phosphine oxides, dialkylsulfoxides and the like. In a particular embodiment, the non-ionic surfactant is selected from ethylene oxide and propylene oxide block copolymers (e.g. poloxamers), ethoxylated hydrogenated castor oils, and mixtures thereof; preferably poloxamer 407, PEG 40 hydrogenated castor oil and mixtures thereof.

Examples of anionic surfactants are alkyl and alkenyl sulfates and their alkyl-ether derivatives, sarcosinates, isethionates and taurates, such as sodium lauryl sulfate, sodium coconut monoglyceride sulfonate, sodium lauryl sarcosinate, sodium lauryl isethionate, sodium laureth carboxylate, salts of C₈-₂₀alkyl sulfates (such as sodium C₁₄-C₁₆ alkyl suflates), salts of C₈-₂₀alkenyl sulfates (such as sodium C₁₄-C₁₆ alkenyl sulfates), and sodium dodecyl benzenesulfonate; preferably sodium lauryl sarcosinate and sodium salts of C₈-₂₀alkyl sulfates (such as sodium C₁₄-C₁₆ alkyl suflates) and sodiumc salts of C₈-₂₀alkenyl sulfates (such as sodium C₁₄-C₁₆ alkenyl sulfates).

Examples of cationic surfactants are lauryl trimethylammonium chloride, cetyl pyridinium chloride, cetyl trimethylammonium bromide, diisobutylphenoxyethyl dimethylbenzylammonium chloride, coconut alkyltrimethylammonium nitrite, cetyl pyridinium fluoride and mixtures thereof.

Examples of amphoteric surfactants are myristyl betaine, palmityl betaine, lauryl betaine, cetyl betaine, stearyl betaine, lauramidopropyl betaine, cocoamidoethyl betaine, cocoamidopropyl betaine and the like; preferably cocamidopropylbetaine.

Preferred surfactants include polyoxyethylene hydrogenated castor oils, poloxamers (polypropylene glycol- polyethylene glycol block copolymers), salts of C₈-₂₀alkyl sulfates, salts of C₈-₂₀alkenyl sulfates, sodium lauryl sarcosinate, and cocoamidopropyl betaine.

One or more surfactants are optionally present in a total amount in the range of from 1 wt% to 70 wt%, wherein the wt% is expressed with respect to the total weight of the composition.

In addition to water, the oral care compositions of the invention may comprise one or more co-solvents such as glycerin, propylene glycol and/or ethanol. One or more co-solvents are optionally present in a total amount in the range of from 5 wt% to 20 wt%, wherein the wt% is expressed with respect to the total weight of the composition.

The oral care compositions of the present invention may optionally comprise a humectant, which is a substance that holds and retains moisture. Any orally acceptable humectant can be used, such as sugar alcohols, e.g. sorbitol and, and low molecular weight PEGs, such as PEG40. Most humectants also function as sweeteners. One or more humectants are optionally present in a total amount in the range of from 1 wt% to 70 wt%, wherein the wt% is expressed with respect to the total weight of the composition.

In addition to the humectants described above and to the glycyrrhizin derivatives described above (which are essential components of the invention), which also function as sweeteners (i.e. substances capable of providing a sweet taste), the oral care compositions of the present invention may optionally comprise a sweetener which is not a humectant. Examples of this type of sweeteners include artificial sweeteners such as sucralose, saccharin, acesulfam, neotam, neohesperidin, cyclamate, thaumatin, stevioside. These sweeteners (which are neither humectants not glycyrrhizin derivatives) may be present in amounts in the range of 0.001 wt% to 2 wt%, wherein the wt% is expressed with respect to the total weight of the composition.

The oral care compositions of the invention may contain a binder, which provides cohesion to the composition. Any conventional binder may be utilized, such as, carboxyvinyl polymers, carrageenans, starches, cellulosic polymers e.g. hydroxyethylcellulose. carboxymethylcellulose (carmellose), hydroxypropyl methyl cellulose and salts thereof (e.g., carmellose sodium), natural gums such as xanthan gum, karaya, gum arabic and tragacanth, and chitosan. One or more binders may be present in a total amount in the range 0.1 wt% to 1.5 wt%, wherein the wt% is expressed with respect to the total weight of the composition.

Mouth-feel agents that may be present in the oral care compositions of the invention and refer to materials which impart a desirable texture or other feeling during use of the composition. Such agents include bicarbonate salts, which may impart a clean feel to teeth and gums due to effervescence and release of carbon dioxide. Any orally acceptable bicarbonate can be used, such as alkali metal bicarbonates e.g. sodium and potassium bicarbonates, ammonium bicarbonate, and mixtures thereof. One or more bicarbonate salts are optionally present in a total amount in the range of 0.1 wt% to 5 wt%, wherein the wt% is expressed with respect to the total weight of the composition.

The oral care compositions of the present invention may also include an abrasive, which removes material from the teeth and gum surfaces and aids in mechanical tooth cleaning. The abrasive may be a silica abrasive such silica, sodium metaphosphate, potassium metaphosphate, tricalcium phosphate, dihydrated dicalcium phosphate, aluminum silicate, calcined alumina, bentonite or other siliceous materials, sodium chloride, or combinations thereof. One or more abrasives may be present in a total amount in the range from 10 wt% to 30 wt%, preferably from 15 wt% to 20 wt% %, wherein the wt% is expressed with respect to the total weight of the composition.

The oral care composition of the present invention may also comprise a desensitizing agents, which treat hypersensitivity by blocking dentin tubules when applied to a tooth.

Examples of desensitizing agents are potassium salts such as potassium nitrate, potassium chloride, potassium bicarbonate, potassium citrate, and potassium oxalate. Such agents may be added in effective amounts, e.g., in a total amount typically in the range from 0.01 wt % to 5 wt%, wherein the wt% is expressed with respect to the total weight of the composition.

In addition to the oral care active ingredients mentioned above (such as zinc salts having a solubility in water higher than 3.0·10⁴ mg/L at 25 °C, isopropylmethylphenols, glycyrrhizin derivatives, fluoride ion sources and desenstizing agents), other oral care active ingredients may be present in the compositions of the invention. Said oral care active ingredients may exert any beneficial effect to teeth and gums, such as reducing hypersensitivity of the teeth, reducing plaque accumulation, reducing or inhibiting demineralization and promote remineralization of the teeth, inhibiting microbial biofilm formation in the oral cavity, reducing or inhibiting gingivitis, promoting healing of sores, reducing levels of acid producing bacteria, increasing relative levels of non-cariogenic and/or non-plaque forming bacteria, reducing or inhibiting formation of dental caries, reducing, repairing or inhibiting pre-carious lesions of the enamel, treating, relieving or reducing dry mouth, cleaning the teeth and oral cavity, reducing erosion, whiten teeth, and reducing tartar build-up. Examples of said agents include alkali metal gluconate salts such as sodium gluconate, panthenol, vitamin E or a derivative thereof such as vitamin E acetate, α-bisabolol, malic acid, calcium glycerophosphate, etc. It is to be understood that the weights of these active ingredients may vary depending on the particular active ingredient, and one of skill in the art may readily determine such amounts. Typically each oral care active ingredient is optionally present in an amount in the range from 0.001 wt% to 1 wt%%, wherein the wt% is expressed with respect to the total weight of the composition.

The oral care compositions of the present invention may comprise a pH modifying agent, which is a compound that adjust and/or maintain pH. Such agents include acidifying agents to lower pH, basifying agents to raise pH, and buffering agents to control pH within a desired range. For example, one or more compounds selected from acidifying, basifying and buffering agents can be included to provide a pH of 4.5 to 10.5, preferably 5.5 to 8.5.

Any orally acceptable pH modifying agent can be used, including carboxylic, phosphoric and sulfonic acids and their salts (e.g., monopotassium phosphate, dipotassium phosphate, monosodium phosphate, trisodium phosphate, citric acid, monosodium citrate, disodium citrate, monosodium malate, etc.), alkali metal hydroxides such as sodium hydroxide, carbonates such as sodium carbonate, bicarbonates, sesquicarbonates, borates, silicates, imidazole and the like. One or more pH modifying agents are optionally present in a total amount effective to maintain the composition in an orally acceptable pH range.

The oral care compositions of the invention may optionally comprise a chelating agent, which forms complexes with metal ions, such as calcium, magnesium and iron. Examples of chelating agents are citric acid, succinic acid, gluconic acid, etidronic acid, galactaric acid, galacturonic acid, glucuronic acid, phytic acid, ethylenediaminetetraacetic acid (EDTA), methylglycine-*N*,*N*-diacetic acid (MGDA), *N*,*N*-Dicarboxymethyl glutamic acid (GLDA), and the respective salts of ammonium, sodium and potassium; poly-acrylic acids, polymers thereof and the respective salts of ammonium, sodium and potassium; and phosphoric acids and poly-phosphoric acids and the respective salts of ammonium, sodium and potassium. Preferably, the chelating agent is EDTA or an ammonium, sodium or potassium salt thereof.. One or more chelating agents are optionally present in a total amount in the range of from 0.1 to 5 wt%, wherein the wt% is expressed with respect to the total weight of the composition.

Preservatives may also be present in the oral care compositions of the invention. Preservatives inhibit the development of microorganism in the oral care composition. Examples of suitable preservatives are chlorhexidine, triclosan, quaternary ammonium compounds (such as benzalkonium chloride) or parabens (such as methyl or propyl paraben). One or more preservatives are optionally present in a total amount typically in the range from 0.001 wt% to 0.5 wt%, wherein the wt% is expressed with respect to the total weight of the composition.

The oral care compositions of the present invention may optionally comprise a flavoring agent that is added to modify the taste and the aroma of the oral care compositions. Flavoring agents that may be used in the compositions of the present invention include essential oils as well as various flavoring aldehydes, esters, alcohols, and similar materials. Examples of the essential oils include oils of spearmint, peppermint, aniseed, wintergreen, sassafras, clove, sage, eucalyptus, marjoram, cinnamon, lemon, lime, grapefruit, and orange. Also useful are compounds such as menthol, carvone, anethole and vainillin. Of these, the most commonly employed are the oils of peppermint and spearmint. One or more flavoring agents may be present in the oral care composition at a total concentration in the range of 0.01 wt% to 5 wt%, wherein the wt% is expressed with respect to the total weight of the composition.

The oral care composition of the invention may comprise at least one colorant, which is added to modify the colour of the compositions. Colorants herein include pigments, dyes, lakes and agents imparting a particular luster or reflectivity such as pearling agents. Any orally acceptable colorant can be used, such as titanium dioxide. One or more colorants are optionally present in a total amount in the range of from 0.0001 wt% to 5 wt%, wherein the wt% is expressed with respect to the total weight of the composition.

The oral care compositions of the invention may be in the form of a mouthwash, a toothpaste or a tooth gel.

In the context of the present invention, "mouthwash" is equivalent to "oral rinse", "mouth rinse" and "mouth bath" and designate liquid compositions which are suitable for oral hygiene, such as by reducing the microbial load in the oral cavity, in particular the bacterial plaque that causes cavities, gingivitis and bad breath. When using such compositions, they are typically maintained in the mouth passively and/or swished through the teeth by contraction of the perioral muscles and/or movement of the head, and may be gargled, and finally spit out.

In the context of the present invention, the term "toothpaste" refers to a semisolid preparation suitable for cleaning the teeth when used with a toothbrush. In the context of the present invention, the term "tooth gel" refers to a gel preparation suitable for cleaning the teeth when used with a toothbrush. Tooth gels are like toothpastes but clear or translucent in appearance. In particular, tooth gels do not comprise titanium dioxide whereas toothpastes comprise titanium dioxide.

In a particular embodiment, the oral care composition of the invention is a mouthwash and comprises water in an amount of at least 70 wt%, at least 75 wt%, or at least 80 wt%; preferably at least 75 wt% of water. The wt% is expressed with respect to the total weight of the composition.

In another particular embodiment, the oral care composition of the invention is a tooth gel or toothpaste and comprises water in an amount of at least at least 10 wt%, at least 15 wt%, at least 20 wt%, at least 25 wt%, at least 30 wt%; preferably at least 10 wt% of water. The wt% is expressed with respect to the total weight of the composition.

In another particular embodiment, the oral care composition of the invention is a tooth gel or toothpaste and comprises water in an amount of from 10 wt% to 35 wt%, from 15 wt% to 35 wt%, from 20 wt% to 35 wt%, from 25 wt% to 35 wt%, from 30 wt% to 35 wt%; preferably from 10 wt% to 35 wt% of water. The wt% is expressed with respect to the total weight of the composition.

In a particular embodiment, the oral care composition of the invention is in the form of a mouthwash and comprises:
- 0.01 to 1.5 wt% of a zinc salt having a solubility in water higher than 3.0·10⁴ mg/L at 25 °C,
- 0.005 to 1 wt% of an isopropylmethylphenol compound,
- 0.005 to 1.5 wt% of a glycyrrhizin derivative selected from the group consisting of glycyrrhizinic acid, glycyrrhetinic acid, salts thereof, and mixtures thereof,
- at least 70 wt% of water,
- optionally 5 to 10 wt% of propylene glycol,
- optionally 1 to 10 wt% of glycerin, and
- optionally 1 to 5 wt% PEG40 hydrogenated castor oil,
wherein the wt% is expressed with respect to the total weight of the composition.

In a preferred embodiment, the oral care composition of the invention is in the form of a mouthwash and comprises:
- 0.01 to 1.5 wt% of zinc chloride,
- 0.005 to 1 wt% of o-cymen-5-ol,
- 0.005 to 1.5 wt% of dipotassium glycyrrhizinate,
- at least 70 wt% of water,
- optionally 5 to 10 wt% of propylene glycol,
- optionally 1 to 10 wt% of glycerin, and
- optionally 1 to 5 wt% PEG40 hydrogenated castor oil,
wherein the wt% is expressed with respect to the total weight of the composition.

In a particular embodiment, the oral care composition of the invention is in the form of a tooth gel or toothpaste, preferably a tooth gel, and comprises:
- 0.01 to 1.5 wt% of a zinc salt having a solubility in water higher than 3.0·10⁴ mg/L at 25 °C,
- 0.005 to 1 wt% of an isopropylmethylphenol compound,
- 0.005 to 1.5 wt% of a glycyrrhizin derivative selected from the group consisting of glycyrrhizinic acid, glycyrrhetinic acid, salts thereof, and mixtures thereof,
- 10 to 35 wt% of water,
- 30 to 55 wt% of sorbitol,
- optionally 0.1 to 1.5 wt% xanthan gum,
- optionally 0.5 to 10 wt% of propylene glycol, and
- optionally 1 to 15 wt% of glycerin,
wherein the wt% is expressed with respect to the total weight of the composition.

In a preferred embodiment, the oral care composition of the invention is in the form of a tooth gel or toothpaste, preferably a tooth gel, and comprises:
- 0.01 to 1.5 wt% of zinc chloride,
- 0.005 to 1 wt% of o-cymen-5-ol,
- 0.005 to 1.5 wt% of dipotassium glycyrrhizinate,
- 10 to 35 wt% of water,
- 30 to 55 wt% of sorbitol,
- optionally 0.1 to 1.5 wt% xanthan gum,
- optionally 0.5 to 10 wt% of propylene glycol, and
- optionally 1 to 15 wt% of glycerin,
wherein the wt% is expressed with respect to the total weight of the composition.

In a particular embodiment, the oral care composition of the invention is in the form of a tooth gel and comprises:
- 0.01 to 1.5 wt% of zinc chloride,
- 0.005 to 1 wt% of o-cymen-5-ol,
- 0.005 to 1.5 wt% of dipotassium glycyrrhizinate,
- 10 to 35 wt% of water,
- 30 to 55 wt% of sorbitol,
- optionally 0.1 to 1.5 wt% xanthan gum,
- optionally 0.5 to 10 wt% of propylene glycol, and
- optionally 1 to 15 wt% of glycerin,
wherein the wt% is expressed with respect to the total weight of the composition.

In another particular embodiment, the oral care composition of the invention is in the form of a toothpaste and comprises:
- 0.01 to 1.5 wt% of zinc chloride,
- 0.005 to 1 wt% of o-cymen-5-ol,
- 0.005 to 1.5 wt% of dipotassium glycyrrhizinate,
- 10 to 25 wt% of water,
- 30 to 40 wt% of sorbitol,
- 0.1 to 1.5 wt% of xanthan gum,
- optionally 0.5 to 5 wt% of propylene glycol, and
- optionally 10 to 15 wt% of glycerin,
wherein the wt% is expressed with respect to the total weight of the composition.

The oral care compositions of the invention may be obtained by mixing the above mentioned ingredients.

### Uses of the compositions

As explained in the background section, zinc salts have antimicrobial properties against different bacteria, which helps to control the development of dental plaque, calculus formation, bleeding gums, and malodour. Zinc salts also reduce the breath concentrations of volatile sulphur compounds (VSC), which are associated with halitosis, as well as having direct and indirect inhibitory effects on anaerobe mediated-VSC production. Also, isopropylmethylphenols have been reported to have high bactericidal effect and to penetrate deep-inside plaque. Further, glycyrrhizin compounds have also been reported as having anti-inflammatory activity which helps in preventing gingivitis and periodontitis [Asl, M. N. et al, Phytother. Res., 2008, 22, 709-724].

Accordingly, a second aspect of the present invention is directed to the oral care compositions of the invention for use in maintaining the health of the oral cavity, in particular for use in the treatment and/or prevention of dental plaque formation, caries formation, calculus formation, halitosis, gingivitis, periodontitis, peri-implant mucositis and/or periimplantitis.

This aspect may also be formulated as the use of an oral care composition of the invention for the manufacture of a medicament for treating and/or preventing dental plaque formation, caries formation, calculus formation, halitosis, gingivitis, periodontitis, peri-implant mucositis and/or periimplantitis.

Alternatively, this aspect may be formulated as a method for the treatment and/or prevention of dental plaque formation, caries formation, calculus formation, halitosis, gingivitis, periodontitis, peri-implant mucositis and/or periimplantitis comprising contacting a surface of the oral cavity with the oral care compositions of the invention.

Alternatively, this aspect may be formulated as the use of the oral care compositions of the invention for the treatment and/or prevention of dental plaque formation, caries formation, calculus formation, halitosis, gingivitis, periodontitis, peri-implant mucositis and/or periimplantitis.

For the above purposes, the aqueous oral composition of the invention may be contacted with a surface of the oral cavity. Said surface of the oral cavity may be selected from the group consisting of one or more teeth, surfaces of the gums, surface of the tongue and combinations thereof. For performing said contacting, when the oral care composition of the invention a mouthwash, it is typically held in the oral cavity passively or swirled around the mouth by contraction of the perioral muscles and/or movement of the head, and may be gargled, and finally spit out. In the case of mouthwashes, the amount of composition dosed in the oral cavity to perform the activity is typically from 2 to 30 ml, preferably from 5 to 20 ml and most preferable from 10 to 15 ml. The contacting may be performed for any suitable amount of time, such as for less than thirty seconds, for thirty seconds or more, for about thirty seconds, for about forty seconds, for about one minute or more. For performing the contact with a surface of the oral cavity, when the oral care composition of the invention is a tooth gel or toothpaste, it is typically applied with a toothbrush and mechanical cleaning of the teeth and/or tongue is performed, preferably for at least two minutes, more preferably for at least three minutes. In the case of tooth gels and toothpastes, the amount of composition dosed to perform the activity is typically from 0.3 to 4.0 g, preferably from 0.5 to 2.5 g. The contacting may be carried out at least once daily, preferably at least twice daily, more preferably at least three times daily, such as 1, 2, 3 or 4 times per day. The oral cavity may belong to a human or animal, preferably a human.

The term "treatment", "treating" or "treat", as used herein, refers to means reversing, alleviating, inhibiting the progress of the disease or disorder these terms refer to, or one or more symptoms of such disease or condition.

The term "prevention", "preventing" or "prevent", as used herein, refers to avoiding the appearance of a certain disease or disorder. The prevention can be complete (e.g. the total absence of a disease or disorder). The prevention can also be partial, such that for example the occurrence of a disease or disorder in a subject is less than that which would have occurred without the administration of the oral care composition of the present invention. The prevention also includes reducing the risk of suffering the disease or disorder.

The term "dental plaque" refers to a fine film comprised of remains of food, bacteria and saliva. If dental plaque is not eliminated regularly, it sticks to the surface of the enamel, attacking tooth structures and producing a series of substances that cause the gum swelling, giving rise to gingivitis (inflammation of the gum), redness and probable gingival bleeding.

The term "calculus" or "tartar" refers to a form of hardened dental plaque. It is caused by precipitation of minerals from saliva and gingival crevicular fluid in plaque on the teeth. This process of precipitation kills the bacterial cells within dental plaque, but the rough and hardened surface that is formed provides an ideal surface for further plaque formation. This leads to calculus buildup, which compromises the health of the gingiva (gums). Calculus can form both along the gumline, where it is referred to as supragingival, and within the narrow sulcus that exists between the teeth and the gingiva, where it is referred to as subgingival.

The term "gingivitis" refers to an inflammation of the gums that gives rise to an increase in sensitivity and irritation and which in many cases is accompanied by bleeding. Its most frequent trigger is dental plaque or biofilm, consisting of a fine film comprised of remains of food, bacteria and saliva. If it is not treated in time, it can evolve to periodontitis (pyorrhoea), and even enter the tooth and damage its bone structure, leading to the irreversible loss of the tooth.

The term "periodontitis" refers to an inflammatory gingival process that spreads to the periodontal ligament which holds and fixes the tooth, and even the alveolar bone, destroying both tissues. Periodontal bags appear, which are an increase in the gap or space between the surface of the tooth and the internal part of the gum. This destructive process spreads until tooth stability is compromised. Due to the loss of fixation, in the end stages the teeth become separated and loose, losing their normal location, and finally the tooth irremediably falls out. *Streptococci*, *spirochetes* and *bacteroides* are found to be the possible pathogens responsible for the disease [Prasanth M., Dent Res J (Isfahan), 2011, 8(2), 85-94; Menendez A. et al., Oral Microbiology Immunology, 2005, 20(1), 31-34].

The term "halitosis" or "bad breath" refers to the presence of unpleasant odours that can be detected in exhaled air. Halitosis is a condition that affects many people. There are multiple types of halitosis and they are classified depending on the source or cause of this bad breath: physiological halitosis, pathological halitosis (oral cause, 87% of all halitosis, and extraoral cause), pseudohalitosis and halitophobia. Bad breath through an oral cause is therefore the most common. This type of halitosis is caused by the production and release of volatile compounds, mainly sulphur compounds. Other molecules such as the diamines (for example, cadaverine) are also related to halitosis. The volatile sulphur compounds are caused by bacteria that grow in the mouth in conditions of oxygen absence (anaerobes). These volatile sulphur compounds come from the metabolism of food bacteria or remain trapped in the mouth.

The term "caries" or "cavities" refers to the breakdown of teeth structure due to acids produced by bacteria. *Streptococcus mutans* is considered one of the main bacteria involved in this process. In addition, other microflora like *Escherichia coli* and *Candida* are also associated with active caries lesions [Prasanth M., Dent Res J (Isfahan), 2011, 8(2), 85-94]. The cavities may be a number of different colors from yellow to black. Symptoms may include pain and difficulty with eating. Complications may include inflammation of the tissue around the tooth, tooth loss, and infection or abscess formation. It is a disease characterized by a series of reactions that lead the teeth's hard tissues to soften, and which if it goes untreated, advances from the surface inwards, eventually leading to the irreversible destruction of the tooth. Caries normally goes unnoticed by the patient. A detailed exploration will detect stains or white chalk-like spots (areas where there is weakened enamel) or brown pigmentation on the tooth surface. This phase may be characterized by a certain sensitivity (strange sensation) to some foods, particularly sweets or hot or cold drinks, although usually there is no actual pain. When the caries advances and affects the pulp or nerve inside the tooth, pain will generally ensue, although if the affection is very slow it may destroy a large part of the nerve painlessly. When the affection is deep, abscesses may form (better known as gumboils, i.e. destroyed dental material and pus) and give rise to major inflammation and pain.

The term "peri-implant mucositis" refers to an inflammatory lesion of the soft tissues surrounding an endo-osseous implant in the absence of loss of supporting bone or continuing marginal bone loss. Peri-implant mucositis is caused by bacteria from dental biofilms, which disrupts the host-microbe homeostasis at the implant-mucosa interface, resulting in an inflammatory lesion. Biofilm removal is a prerequisite for the prevention and management of peri-implant mucositis. Peri-implant mucositis is considered a precursor for peri-implantitis.

The term "periimplantitis" refers to an inflammatory lesion of the mucosa that affects the surrounding tissue of the implant, predominately the supporting bone with loss of osseointegration. As peri-implant mucositis, it is also caused by the adhesion of pathogenic bacterial biofilms on the implant surface and peri-implant tissues.

In the context of the invention, the term "about" when characterizing a value refers to ±10% of said value, preferably ±5% of said value.

Preferably, the expression "one or more" refers to 1, 2, 3, 4, 5, 6, 7, 8, 9 or 10, more preferably to 1, 2, 3, 4 or 5, even more preferably to 1, 2 or 3.

The following examples are provided to illustrate the invention but should not be considered as limiting its scope.

### Examples

### Compositions

Oral care compositions (200 mL) were prepared by mixing the ingredients disclosed in the table below. The ingredients and their amounts (expressed as %w/w, i.e. wt%) of each composition is provided in the table below.

| *Ingredient* | *Comparative example 1* | *Comparative example 2* | *Example 3* | *Example 4* |
|---|---|---|---|---|
| ZnCl₂ | 0.1 | 0.1 | 0.1 | 0.1 |
| Dipotassium glycyrrhizinate | 0 | 0 | 0.1 | 0.1 |
| *o*-Cymen-5-ol | 0 | 0.1 | 0.1 | 0.1 |
| Propylene glycol | 7 | 7 | 7 | 7 |
| PEG40 hydrogentated castor oil | 2 | 2 | 2 | 2 |
| Xanthan gum | 0 | 0 | 0 | 1 |
| Water | *q.s.* 100 | *q.s.* 100 | *q.s.* 100 | *q.s.* 100 |
| pH | 5.4 | 5.83 | 5.6 | - |

The tables below disclose specific mouthwash, toothpaste and tooth gel formulations.

**Mouthwash example formulation 5:**

| *Ingredient* | *%w*/*w* |
|---|---|
| o-Cymen-5-ol | 0.10 |
| Dipotassium glycyrrhizinate | 0.05 |
| Zinc chloride | 0.10 |
| Sodium fluoride | 0.31 |
| Potassium nitrate | 3.00 |
| Glycerine | 3.00 |
| Propylene glycol | 7.00 |
| PEG 40 hydrogenated castor oil | 2.00 |
| Panthenol | 0.25 |
| Chelating agents | 1.00 |
| Preservatives | 0.50 |
| Sweeteners | 1.00 |
| Binder | 0.20 |
| Flavoring agent | 0.20 |
| Water | 81.29 |
| Total | 100.00 |

**Mouthwash example formulation 6:**

| *Ingredient* | *%w*/*w* |
|---|---|
| o-Cymen-5-ol | 0.10 |
| Dipotassium glycyrrhizinate | 0.10 |
| Zinc chloride | 0.10 |
| Sodium fluoride | 0.31 |
| Glycerine | 3.00 |
| Propylene glycol | 7.00 |
| PEG 40 hydrogenated castor oil | 2.00 |
| Panthenol | 0.25 |
| Chelating agents | 1.00 |
| Preservatives | 0.50 |
| Sweeterners | 1.00 |
| Binder | 0.20 |
| Flavoring agent | 0.20 |
| Water | 84.24 |
| Total | 100.00 |

**Toothpaste example formulation 7:**

| *Ingredient* | *%w*/*w* |
|---|---|
| o-Cymen-5-ol | 0.10 |
| Dipotassium glycyrrhizinate | 0.20 |
| Zinc chloride | 0.60 |
| Sodium fluoride | 0.31 |
| Sorbitol | 43.00 |
| Glycerine | 12.00 |
| Propylene glycol | 2.00 |
| Silicates | 18.50 |
| Titanium dioxide | 1.00 |
| Surfactants | 3.50 |
| Sweeterners | 1.00 |
| Binder | 0.80 |
| Flavoring agent | 1.00 |
| Water | 15.99 |
| Total | 100.00 |

**Tooth gel example formulation 8:**

| Ingreedient | %w/w |
|---|---|
| o-Cymen-5-ol | 0.10 |
| Dipotassium glycyrrhizinate | 0.20 |
| Zinc chloride | 0.60 |
| Sodium fluoride | 0.31 |
| Sorbitol | 43.00 |
| Glycerine | 12.00 |
| Propylene glycol | 2.00 |
| Silicates | 18.50 |
| Surfactants | 3.50 |
| Sweeterners | 1.00 |
| Binder | 0.80 |
| Flavoring agent | 1.00 |
| Water | 16.99 |
| Total | 100.00 |

### Turbidity

After preparation the turbidity of the compositions of Examples 1-3 was assessed by visual inspection (12 hours after preparation) and by nephelometry.

The nephelometry method is based upon a comparison of the intensity of light scattered by the sample under defined conditions with the intensity of light scattered by a reference standard. The higher the intensity of scattered light, the higher the turbidity. Readings, in nephelometric turbidity units (NTU), are made in a Cyberscan IR Turbidimeter TB 1000, using about 30 mL of each test composition. The control was a composition comprising 2 wt% of PEG 40 hydrogenated castor oil, 7 wt% propylene glycol and water (*q.s.* 100 wt%).

The results are gathered in the table below.

| | *Visual inspection* | *Turbidity (NTU)* |
|---|---|---|
| Control | Clear solution | 3 |
| Comparative example 1 | Slight turbidity | 6.06 |
| Comparative example 2 | Slight turbidity | 6.46 |
| Example 3 | Clear solution | 3.52 |

The results show that zinc chloride provides unsatisfactory compositions in terms of turbidity in comparative examples 1 and 2. However, the composition of Example 3, which comprises dipostassium glycyrrhizinate has surprisingly improved the solubility of zinc chloride.

### Microbiological studies

Studies of antimicrobial activity against *Streptococcus mutans* (ATCC 25175), *Escherichia coli* (ATCC 25922) and *Candida albicans* (ATCC 90028) were carried out with the compositions of Comparative Example 1, Comparative Example 2 and Example 3. The control was a composition comprising 2 wt% of PEG 40 hydrogenated castor oil, 7 wt% propylene glycol and water (*q.s.* 100 wt%).

Culture media and incubation conditions for each microorganism are provided in the table below:

| *Microorganism* | *Culture media* | *Incubation conditions* |
|---|---|---|
| *S. mutans* | Trypticase Soy Agar (TSA)/Trypticase Soy Broth (TSB)/Müller-Hinton Broth | 37 °C, 5% CO₂, 48 h |
| *E. coli* | Trypticase Soy Agar (TSA)/Trypticase Soy Broth (TSB) | 37 °C, aerobiosis, 24 h |
| *C. albicans* | Trypticase Soy Broth (TSB)/Sabouraud-Cloramfenicol/RPMI 1640 + 0.2% glucose | 30 °C, aerobiosis, 24-48 h |

### Determination of maximum inhibitory dilution (MID):

Various dilutions of the compositions under study were incubated with the microorganisms (10⁴ cfu/mL) cultured in microtiter plates in the corresponding culture media and incubation conditions. The MID was defined as the highest dilution (i.e. lowest concentration) capable of totally inhibiting visible microbial growth. [EUCAST (European Committee on Antimicrobial Susceptibility Testing) and CLSI (Clinical and Laboratory Standards Institute) guidelines]

### Determination of maximum bactericidal dilution (MBD):

20 µL of the dilutions of the compositions under study from the MID experiment described above where no visible microorganism growth had been observed in the MID assay were seeded in a solid agar medium. The seeded plates were incubated under the conditions described in the table above. The MBD corresponded to the highest dilution (i.e. lowest concentration) capable of totally inhibiting microbial growth.

### Determination of bacterial death kinetics:

Bacterial death kinetics of the compositions under study was determined by contacting suspensions of each type of bacteria at a concentration of from 5x10⁵ to 2x10⁶ cfu/mL. After 1, 2 and 5 minutes a sample (100 µL) was extracted and diluted 100 times. The number of viable cells was determined by seeding in solid agar medium. The plates were incubated under the corresponding conditions provided in the table above and the number of bacteria was determined.

The results of MID and MBD are provided in the Table below, expressed as the dilution of the composition tested.

| | *S. mutans* | | *C. albicans* | | *E. coli* | |
|---|---|---|---|---|---|---|
| | MID | MBD | MID | MBD | MID | MBD |
| Control | ≥ 1/2 | ≥ 1/2 | ≥ 1/2 | ≥ 1/2 | ≥ 1/2 | ≥ 1/2 |
| Comparative Example 1 | 1/2 | 1/2 | 1/16 | 1/2 | 1/2 | 1/2 |
| Comparative Example 2 | ≥ 1/2 | ≥ 1/2 | ≥ 1/2 | ≥ 1/2 | ≥ 1/2 | ≥ 1/2 |
| Example 3 | 1/4 | 1/2 | 1/32 | ≥ 1/2 | 1/2 | 1/2 |

As it can be seen, the composition of Example 3 had the highest bacteriostatic activity and the composition of comparative Example 2 had no activity against tested bacteria.

The compositions having activity (comparative Example 2 and Example 3) were tested to determine their bacterial death kinetics. The results are provided in the Table below expressed as bacterial counts (cfu/mL) and % of survival in parentheses.

| | *C. albicans* | | *S. mutans* | | *E. coli* | |
|---|---|---|---|---|---|---|
| | Comparative Example 1 | Example 3 | Comparative Example 1 | Example 3 | Comparative Example 1 | Example 3 |
| 0 min | 5.00·10⁵ (100%) | 5.00·10⁵ (100%) | 6.00·10⁵ (100%) | 6.00·10⁵ (100%) | 2.00·10⁶ (100%) | 2.00·10⁶ (100%) |
| 1 min | 2.00·10⁴ (4%) | 5.00·10³ (1%) | 5.00·10⁵ (83.33%) | 8.00·10⁵ (>100%) | 2.00·10⁶ (100%) | 1.00·10⁵ (5%) |
| 2 min | 1.00·10⁴ (2%) | 1.00·10⁴ (2%) | 3.00·10⁵ (50%) | 3.50·10⁵ (58.33%) | 5.00·10⁴ (2.5%) | 5.00·10⁴ (2.5%) |
| 5 min | 5.00·10³ (1%) | 1.50·10⁴ (3%) | 5.00·10⁵ (83.33%) | 5.00·10⁵ (83.33%) | 2.00·10⁴ (1%) | 5.50·10⁴ (2.75%) |

As it can be seen, the composition of Example 3 is faster (active at 1 min) against C. *albicans* and *E. coli* than the composition of comparative Example 1.

## Claims

1. Oral care composition comprising:
- a zinc salt having a solubility in water higher than 3.0·10⁴ mg/L at 25 °C,
- an isopropylmethylphenol compound,
- a glycyrrhizin derivative selected from the group consisting of glycyrrhizinic acid, glycyrrhetinic acid, salts thereof, and mixtures thereof, and
- water.

2. Oral care composition according to claim 1, wherein the zinc salt having a solubility in water higher than 3.0·10⁴ mg/L at 25 °C is selected from the group consisting of zinc chloride, zinc sulphate, zinc gluconate, zinc citrate, zinc acetate and mixtures thereof.

3. Oral care composition according to claim 2, wherein the zinc salt having a solubility in water higher than 3.0·10⁴ mg/L at 25 °C is zinc chloride.

4. Oral care composition according to any of the preceding claims, wherein the isopropylmethylphenol compound is o-cymen-5-ol.

5. Oral care composition according to any of the preceding claims, wherein the glycyrrhizin derivative is glycyrrhizinic acid or a salt thereof, preferably dipotassium glycyrrhizinate.

6. Oral care composition according to any of the preceding claims, wherein the zinc salt having a solubility in water higher than 3.0·10⁴ mg/L at 25 °C is present in a concentration of from 0.01 to 1.5 wt% with respect to the total weight of the composition.

7. Oral care composition according to any of the preceding claims, wherein the glycyrrhizin derivative is present in a concentration of from 0.005 to 1.5 wt% with respect to the total weight of the composition.

8. Oral care composition according to any of the preceding claims, wherein the isopropylmethylphenol compound is present in a concentration of from 0.005 to 1 wt% with respect to the total weight of the composition.

9. Oral care composition according to any of the preceding claims, wherein water is present in an amount of at least 10 wt%, at least 15 wt%, at least 20 wt%, at least 25 wt%, at least 30 wt%, at least 40 wt%, at least 50 wt%, at least 60 wt%, at least 70 wt%, at least 75 wt%, or at least 80 wt%, wherein the wt% is expressed with respect to the total weight of the composition.

10. Oral care composition according to any of the preceding claims further comprising one or more ingredients selected from the group consisting of fluoride ion sources, surfactants, co-solvents, humectants, sweeteners, binders, mouth-feel agents, abrasives, desensitizing agents, other oral care active ingredients, pH modifying agents, preservatives, chelating agents, flavoring agents, and colorants.

11. Oral care composition according to any of the preceding claims in the form of a mouthwash, a tooth gel or a toothpaste.

12. Oral care composition according to any of the preceding claims in the form of a mouthwash which comprises:
- 0.01 to 1.5 wt% of zinc chloride,
- 0.005 to 1 wt% of o-cymen-5-ol,
- 0.005 to 1.5 wt% of dipotassium glycyrrhizinate,
- at least 70 wt% of water,
- optionally 5 to 10 wt% of propylene glycol,
- optionally 1 to 10 wt% of glycerin, and
- optionally 1 to 5 wt% PEG40 hydrogenated castor oil,
wherein the wt% is expressed with respect to the total weight of the composition.

13. Oral care composition according to any of claims 1 to 11 in the form of a tooth gel or toothpaste which comprises:
- 0.01 to 1.5 wt% of zinc chloride,
- 0.005 to 1 wt% of o-cymen-5-ol,
- 0.005 to 1.5 wt% of dipotassium glycyrrhizinate,
- 10 to 35 wt% of water,
- 30 to 55 wt% of sorbitol,
- optionally 0.1 to 1.5 wt% xanthan gum,
- optionally 0.5 to 10 wt% of propylene glycol, and
- optionally 1 to 15 wt% of glycerin,
wherein the wt% is expressed with respect to the total weight of the composition.

14. Oral care composition according to any of the preceding claims, wherein the weight ratio of the zinc salt having a solubility in water higher than 3.0·10⁴ mg/L at 25 °C to the glyzyrrhizin derivative is from 5:1 to 1:1.

15. Oral care composition according to any of the preceding claims for use in the treatment and/or prevention of dental plaque formation, caries formation, calculus formation, halitosis, gingivitis, periodontitis, peri-implant mucositis and/or periimplantitis.
